# EUROPEAN PATENT SPECIFICATION

(11) **EP 2 747 678 B1**
(45) Date of publication and mention of the grant of the patent: **08.07.2015**
(21) Application number: 12740403.6
(22) Date of filing: 29.05.2012
(51) Int. Cl.: A61B 17/34

(54) **A TROCAR WITH ENHANCED MANOEUVRE CAPABILITY**
TROKAR MIT VERBESSERTER MANÖVRIERFÄHIGKEIT
TROCART À CAPACITÉ DE MANOEUVRE AMÉLIORÉE

(30) Priority: 26.08.2011 TR 201108671
(43) Date of publication of application: 02.07.2014
(73) Proprietor: Koc Universitesi, 34450 Istanbul (TR)
(72) Inventor: CANADINC, Demircan, 34450 Istanbul (TR); CARILLI, Senol Ibrahim, Istanbul (TR)
(74) Representative: Dericioglu Kurt, Ekin
(86) International application number: PCT/IB2012/052677
(87) International publication number: WO 2013/030682

(56) References cited:
- EP-A2- 0 873 721
- WO-A1-2005/092217
- WO-A1-2005/102186
- DE-A1- 4 210 984
- FR-A1- 2 727 849
- GB-A- 2 315 021
- US-A1- 2005 004 512
- US-A1- 2007 232 988
- US-A1- 2009 192 466

## Description

### Field of the Invention

The present invention relates to a trocar which mediates the surgical instruments used during laparoscopic and other minimal invasive surgeries to be inserted inside the body.

### Background of the Invention

Surgical instruments such as camera, scissors used during closed surgeries are inserted inside the abdominal cavity through the apparatus called trocar. The trocars placed inside the incisions made on the abdominal wall have various valve mechanisms in order to prevent the gases constituting the intra-abdominal pressure from going out of the body. Valve mechanisms are one of the indispensable parts of the trocars in order to maintain the body pressure. However, in today's trocars the entry part of the trocar wherein the valve mechanism is mounted has larger outer diameter than the body part of the trocar which is inserted inside the body since the valve mechanism has a complex structure. The outer diameters of the entry parts of the conventional trocars being larger than the body part causes various problems. One of the biggest problems of the conventional trocars is the manoeuvre loss in laparoscopic operation instruments especially when they are placed at close points to each other. The conventional trocars which are placed from close points to each other cannot enter inside the body parallel to each other since the large outer diameters originating from the valve mechanisms at the parts wherein the surgical instruments are inserted contact each other. Most of the time the ends of the trocars inside the body come close to each other too much or are positioned diagonal to each other. This situation makes it difficult to control the surgical instrument from the part out of the body and the part inside the body to reach the targeted areas.

Another negative side of current trocars is that they do not have a flexible structure since they are generally produced from a material such as hard plastic or metal. Only the surgical instruments having a straight structure can pass through the trocars produced from a non-flexible material. Surgical instruments having a curvy structure cannot pass through the conventional trocars.

Another negative aspect of the conventional trocars is that the hose coming out of the air inlet channel extends towards the hand of the operator, the person who performs the surgery, and disturbs the hand of the operator. In the trocars in the state of the art, air inlet channel is positioned such that it will be towards the end of the trocar entering inside the body or perpendicular to the axis of the trocar in order that it will not cause failure by the air sent inside the trocar with pressure to hit the valve system. For this reason, the hose attached to the air inlet channel in order to transfer air to the body from the air inlet channel extends towards the hand of the operator and disturbs the operator because of the physical contact.

United States Patent document US 5,584,847, an application known in the state of the art, discloses a trocar with a rotating tip wherein the endoscopy instruments can enter.

United States Patent document US 2009/0204140 A1, another application known in the state of the art, discloses adding an environmental protector in order to prevent the trocar from entering more by accident after the puncture.

United States Patent Document US 2007/232988 A1 discloses a trocar with the features according to the preamble of independent claim 1.

### Summary of the Invention

The objective of the present invention is to provide a trocar which prevents the air hose attached to the air inlet channel from extending towards the hand of the person who is operating.

A further objective of the present invention is to provide a trocar which enhances the manoeuvre capability of the surgical instruments by means of its part entering inside the body having almost the same outer diameter with its part outside the body.

Another objective of the present invention is to provide a trocar which enables the curved surgical instruments used in operations to be inserted inside the body in closed operations.

Yet another object of the present invention is to provide a trocar having a body which prevents slipping inside the body.

### Detailed Description of the Invention

"A trocar" realized to fulfill the objectives of the present invention is illustrated in the accompanying figures, in which:

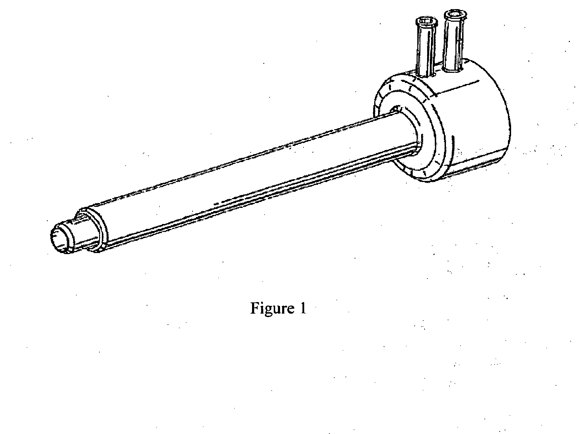

Figure 1 is the view of a trocar in the state of art.

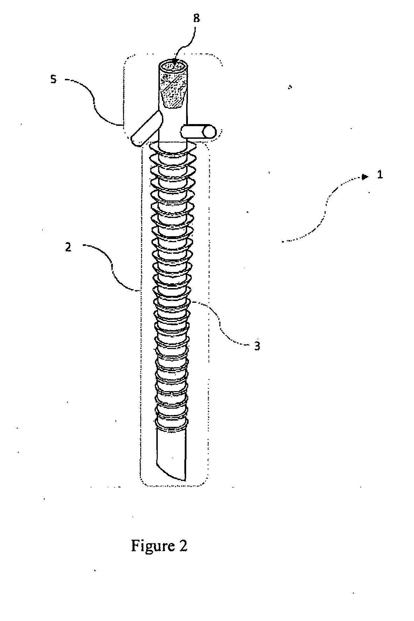

Figure 2 is the view of a trocar the valve part of which is made thin.

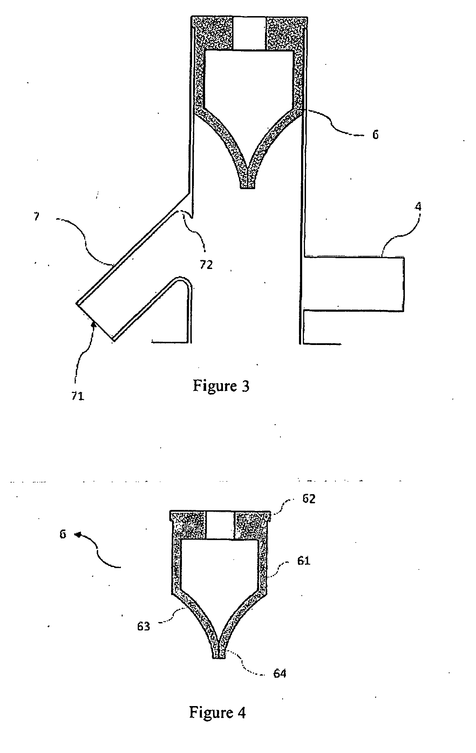

Figure 3 is the cross sectional detailed view of the upper body of the trocar.
Figure 4 is the sectional view of the valve.
Figure 5 is the longitudinal sectional view of the trocar.

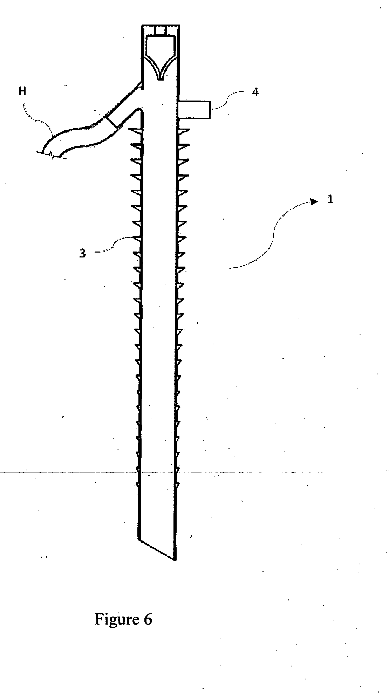

Figure 6 is the sectional view of the trocar wherein the hose is connected to its air inlet-outlet channel.

The components in the figures are numbered individually, where the numbers refer to the following:
1. Trocar
2. Lower body
3. Thread
4. Support
5. Upper body
6. Valve
   61. Valve body
   62. Holder
   63. Valve tip
   64. Groove
7. Air inlet-outlet channel
   71. Opening
   72. Air directing bevel
8. Upper hole
9. Lower hole
10. Drilling tip

A trocar with enhanced manoeuvre capability (1), which is used in various laparoscopic and other minimal invasive surgeries and through which the instruments and/or devices to be used during operation and/or surgery are passed and are inserted inside the body, comprises
- at least one lower body (2) which enters inside the body,
- at least one thread (3) which is located on the lower body (2) and prevents the lower body (2) from slipping inside the body or outside the body during operation,
- at least one support (4) which prevents the lower body (2) from completely entering inside the body,
- at least one upper body (5) which is located at the upper part of the lower body (2) and has the same or almost the same outer diameter with the lower body (2) by means of using a simple valve (6) system,
- at least one valve (6) which is located inside the upper body (5) and prevents air leakage from the body cavity during surgery,
- at least one air inlet-outlet channel (7) which is located on the upper body (5) and enables the pressurized gas to be supplied inside the body or the gasses to be released from the body in order to maintain the body pressure,
- at least one upper hole (8) which enables the devices used during operation to be inserted into the upper body (5) and is located at the end of the upper body (5) on which the lower body (2) is not located,
- at least one lower hole (9) which is located at the end of the lower body (2) entering the body and enables the surgical instruments inserted inside the upper body (5) via the upper hole (8) and then moving through the lower body (2) to go out of the lower body (2) and move inside the body cavity, and
- at least one drilling tip (10) which is located at the end of the lower body (2) entering the body and enables the lower body (2) to be inserted into the body cavity by drilling the abdominal wall.

The inventive trocar (1) comprises an air inlet-outlet channel (7) the end of which not connected to the upper body (5) extends towards the lower body (2).

The lower body (2) preferably has a hollow cylindrical structure and it is the part of the inventive trocar with enhanced manoeuvre capability (1) which goes inside the body. In the preferred embodiment of the invention, there are preferably circular threads (3) on the outer surface of the lower body (2) such that they will be co-axial with the lower body (2). The threads (3) enable the lower body (2) to remain in desired depth while the lower body (2) is inserted through the incisions made on the abdominal wall. By means of the threads (3) the trocar (1) is prevented from moving outwards or inwards or slipping on the abdominal wall unintentionally during the operation. In one embodiment of the invention, each one of the threads (3) has larger outer diameter than the previous thread (3) such that it will have the smallest outer diameter at the end of the lower body (2) entering the body and the largest outer diameter at the end of the lower body (2) connecting with the upper body (5). In one embodiment of the invention, the lower body (2) is produced from any metal or metal alloy. In one embodiment of the invention the lower body (2) is produced from medical grade polypropylene or a flexible material such as medical grade polyethylene. In a preferred embodiment of the invention, by means of producing the lower body (2) from a flexible material, nonlinear instrument can also be inserted inside the body via trocar (1).

The support (4) is located at the part of the lower body (2) which does not enter inside the body. The support (4) prevents the inventive trocar (1) to move through the body further by leaning on the abdominal wall. By this means the trocar (1) is prevented from falling into the body cavity by mistake. In the preferred embodiment of the invention the support (4) is an elliptic structure. In other embodiments of the invention, the support (4) can be circle, triangle, rectangle or geometrical form having more edges or can be a curve. It is possible to develop support (4) embodiments in many more ways.

The upper body (5) is preferably a cylindrical structure one end of which is connected to the end of the lower body (2) that does not enter inside the body. In a preferred embodiment of the invention, the upper body (5) is produced one-piece with the lower body (2). The outer diameter of the upper body (5) is almost same with the outer diameter of the lower body (2). In another embodiment of the invention the outer diameter of the upper body (5) is a bit larger than the outer diameter of the lower body (2). In one embodiment of the invention the upper body (5) is produced from flexible material such as medical grade polypropylene or a medical grade polyethylene.

There is at least one valve (6) present inside the upper body (5) in order to prevent the air inside the body to be released outside during surgery. In the preferred embodiment of the invention, the valve (6) has a hollow cylindrical valve body (61). The valve body (61) engages inside the upper body (5) such that it will fit tightly. The inner diameter of the upper body (5) and the outer diameter of the valve body (61) are in contact with each other and air-sealed. On one of the circular surfaces on the valve body (61), there is a holder (62) which prevents the valve (6) to move towards the lower body (2) inside the upper body (5). The holder (62) has a hollow cylindrical structure. The inner diameter of the holder (62) has preferably same size with the inner diameter of the valve body (61). The outer diameter of the holder (62) has preferably same size with the outer diameter of the upper body (5). On the surface of the valve body (61) on which the holder (62) is not located, that is the surface which is the circular, inserted inside the upper body, there is a valve tip (63). The valve tip (63) is preferably in the shape of a hollow cone. The part of the conical valve tip (63) having the largest outer diameter and thus the inner diameter is adjacent to the circular surface of the valve body (61) entering into the upper body (5). The largest outer diameter of the valve tip (63) is equal to the outer diameter of the valve body (61), and the largest inner diameter of the valve tip (63) is equal to the inner diameter of the valve body (61). The valve tip (63) is produced from a flexible material such as pharmaceutical rubber. On the part of the valve tip (63) farthest to the valve body (61), that is the pointed, top part of the conical valve tip (63), there is at least one groove (64) present in order to allow the instruments to be used during surgery to pass. When the groove (64) is free, that is no instrument is passing through it, it is air-sealed. When an instrument is passed through the groove (64), the valve tip (63) having a flexible structure sticks to the surfaces of the instrument with the effect of the pressurized air and prevents the air from being released. By means of the valve (6) having a simple structure, the outer diameter of the upper body (5) can be produced in almost same size with the outer diameter of the lower body (2).

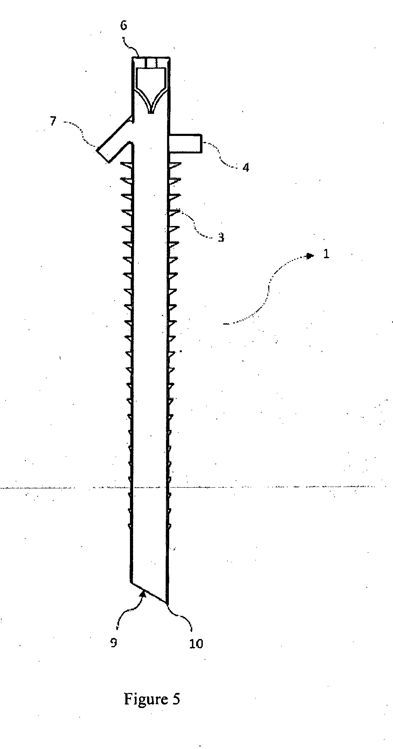

The air inlet-outlet channel (7) is preferably a cylindrical and hollow structure which is located at any place between the valve (6) and the lower body (2) on the upper body (5), and enables the air to be sent to the body cavity during operation or to be released from the body cavity. One end of the air inlet-outlet channel (7) is connected to the upper body (5) and is preferably produced in one-piece. By means of the opening (71) located at the end of the air inlet-outlet channel (7) which is not connected to the upper body (5) gas is received from an external pressurized gas source and the trocar (1) is moved towards the body cavity. In the embodiment of the invention the end of the air inlet-outlet channel (7) which is not connected to the upper body (5) extends towards the lower body (2) (Figure 5). By this means, the hose (H) which is coming from the external air source and connected to the opening (71) of the air inlet-outlet channel (7) extends towards the body of the person who is being operated, not towards the upper part (U arrow direction) of the upper body (5). Therefore, it does not extend towards the upper hole (8) in which the instruments used during surgeries are inserted. The opening (71) part of the air inlet-outlet channel (7) being designed as it extends towards the lower body (2) causes the air sent through the channel (7) to hit the valve (6) first. The air sent with high pressure directly hitting the valve (6) may cause the valve (6) to be harmed and not to function. In order to prevent the air from hitting directly to the valve (6), the part of the air inlet-outlet channel (7) connecting with the upper body (5) is preferably produced inclined towards the lower body (2). The air directing bevel (72) prevents the air coming with high pressure to hit directly to the valve (6). In one embodiment of the invention the half of the cylindrical surface of the air inlet-outlet channel (7) connecting with the upper body (5) which is close to the valve (6) is inclined towards the lower body (2) and thus the air directing bevel (72) is constituted.

It is possible to develop various embodiments of the inventive "TROCAR WITH ENHANCED MANOUEVRE CAPABILITY" (1). The invention can not be limited to the examples described herein and the invention is defined in the appended claims.

## Claims

1. A trocar (1) which is used in various laparoscopic and other minimal invasive surgeries and through which the instruments and/or devices to be used during operation and/or surgery are passed and are inserted inside the body **comprising**
- at least one body (2) which enters inside the body,
- at least one thread (3) which is located on the lower body (2) and prevents the lower body (2) from slipping inside the body or outside the body during operation,
- at least one support (4) which prevents the lower body (2) from completely entering inside the body,
- at least one upper body (5) which is located at the upper part of the lower body (2) and has the same or almost the same outer diameter with the lower body (2) by means of using a simple valve (6) system,
- at least one valve (6) which is located inside the upper body (5) and prevents air leakage from the body cavity during surgery,
- at least one air inlet-outlet channel (7) which is located on the upper
- body (5) and enables the pressurized gas to be supplied inside the body or the gasses to be released from the body in order to maintain the body pressure,
- at least one upper hole (8) which enables the devices used during operation to be inserted into the upper body (5) and is located at the end of the upper body (5) on which the lower body (2) is not located,
- at least one lower hole (9) which is located at the end of the lower body (2) entering the body and enables the surgical instruments inserted inside the upper body (5) via the upper hole (8) and then moving through the lower body (2) to go out of the lower body (2) and move inside the body cavity, and
- at least one drilling tip (10) which is located at the end of the lower body (2) entering the body and enables the lower body (2) to be inserted into the body cavity by drilling the abdominal wall, and **characterized by**
- an air inlet-outlet channel (7) the end of which not connected to the upper body (5) extends towards the lower body (2)

2. A trocar (1) according to claim 1, **characterized by** threads (3) which are circular such that they will be co-axial with the lower body (2).

3. A trocar (1) according to any one of the preceding claims, **characterized by** threads (3) which have larger outer diameter than the previous thread (3) such that it will have the smallest outer diameter at the end of the lower body (2) entering the body and the largest outer diameter at the end of the lower body (2) connecting with the upper body (5).

4. A trocar (1) according to any one of the preceding claims, **characterized by** lower body (2) which is produced from a flexible material that allows the nonlinear instruments to be inserted inside the body via trocar (1).

5. A trocar (1) according to any one of the preceding claims, **characterized by** a support which has an elliptic form (4).

6. A trocar (1) according to any of the preceding claims **characterized by** upper body (5) which is produced in one-piece with the lower body (2).

7. A trocar (1) according to any of the preceding claims, **characterized by** valve (6) comprising -a valve body (61) which is in the shape of a hollow cylinder,
- a support (62) which prevents the valve (6) from moving towards the lower body (2),
- a valve tip (63) which is conical and located at the end of the valve (6) being inserted inside the body (5),
- a groove (64) which is located on the valve tip (63) in order to allow the instruments to be used during surgeries to pass.

8. A trocar (1) according to Claim 7, **characterized by** valve tip (63) which is produced from a flexible material.

9. A trocar (1) according to any one of the preceding claims, **characterized by** air directing bevel (72) which is constituted towards the lower body (2) on the part of the air inlet-outlet channel (7) connecting with the upper body (5) in order to prevent the air sent from the air inlet-outlet channel from hitting directly to the valve (6).

10. A trocar (1) according to any of the preceding claims, **characterized by** lower body (2) which is produced from metal or metal alloy.

11. A trocar (1) according to any one of the preceding claims, **characterized by** lower body (2) which is produced from medical grade polypropylene or medical grade polyethylene.

12. A trocar (1) according to any one of the preceding claims, **characterized by** a valve tip (63) which is produced from pharmaceutical rubber.

13. A trocar (1) according to any of the preceding claims **characterized by** upper body (5) which is produced medical grade polypropylene or medical grade polyethylene.

## Patentansprüche

1. Ein Trokar (1), welcher in verschiedenen laparoskopischen und anderen minimalinvasiven Chirurgie angewendet wird und durch welchen die während der Operation und/oder Chirurgie zu verwendenden Instrumente und/oder Geräte durchgeführt und in den Körper eingesetzt werden, **umfassend**
- mindestens ein Gehäuse (2), das in den Körper eindringt,
- mindestens ein Gewinde (3), das auf dem unteren Gehäuse (2) angeordnet ist und es verhindert, dass das untere Gehäuse (2) während der Operation innerhalb des Körpers oder ausserhalb des Körpers rutscht,
- mindestens eine Stütze (4), welche das untere Gehäuse (2) verhindert völlig in den Körper einzudringen,
- mindestens ein oberes Gehäuse (5), der auf dem oberen Teil des unteren Gehäuses (2) angeordnet ist und den gleichen bzw. fast gleichen Durchmesser wie das untere Gehäuse (2) aufweist, wobei ein einfaches Ventil (6)-System verwendet wird,
- mindestens ein Ventil (6), das innerhalb des oberen Gehäuses (5) angeordnet ist und während der Chirurgie den Luftleckverlust aus dem Körper-Vertiefung verhindert,
- mindestens einen Lutfein-und -ausgangskanal (7), der auf dem oberen Gehäuse (5) angeordnet ist und es ermöglicht, dass das Druckgas in den Körper zugeführt wird oder die Gase aus dem Körper ausgelassen werden, um den Körperdruck aufrechtzuerhalten,
- mindestens eine obere Öffnung (8), die es ermöglicht, dass die während der Operation verwendeten Geräte in das obere Gehäuse (5) eingesetzt werden und am Ende des oberen Gehäuses (5) angeordnet ist, auf welchem das untere Gehäuse (2) nicht angeordnet ist,
- mindestens eine untere Öffnung (9), die am Ende des unteren Gehäuses (2) angeordnet in den Körper eindringt und es ermöglicht, dass die innerhalb des unteren Gehäuses (5) eingesetzten chirurgischen Instrumente über die obere Öffnung (8) und dann durch das untere Gehäuse (2) hindurch aus dem unteren Gehäuse (2) austreten und sich in die Körper-Vertiefung bewegen und
- mindestens eine Bohrspitze (10), die am Ende des unteren Gehäuses (2) angeordnet in den Körper eindringt und es ermöglicht, dass das untere Gehäuse (2) durch Bohren der Bauchwand in die Körper-Vertiefung eingesetzt wird und **gekennzeichnet durch**
- einen Luftein-und-ausgangskanal (7), dessen Ende sich ungebunden mit dem oberen Gehäuse (5) gegen das untere Gehäuse (2) erstreckt.

2. Ein Trokar (1) gemäß Anspruch 1, **gekennzeichnet durch** die Gewinde (3), welche kreisförmig sind, so dass sie mit dem unteren Gehäuse (2) koaxial sein werden.

3. Ein Trokar (1) gemäß einem der vorangehenden Ansprüche, **gekennzeichnet durch** die Gewinde (3), welche größere Aussendurchmesser haben als das vorangehende Gewinde (3), so dass es am Ende des in den Körper eindringenden unteren Gehäuses (2) den kleinsten Aussendurchmesser sowie am Ende des mit dem oberen Gehäuse (5) verbundenen unteren Gehäuses (2) den größten Aussendurchmesser aufweist.

4. Ein Trokar (1) gemäß einem der vorangehenden Ansprüche, **gekennzeichnet durch** das untere Gehäuse (2), das aus einem nachgiebigen Material hergestellt ist, welches es ermöglicht, dass die nichtlinearen Instrumente über Trokar (1) innerhalb des Körpers eingesetzt werden.

5. Ein Trokar (1) gemäß einem der vorangehenden Ansprüche, **gekennzeichnet durch** eine Stütze, welche eine elliptische Form (4) aufweist.

6. Ein Trokar (1) gemäß einem der vorangehenden Ansprüche, **gekennzeichnet durch** ein oberes Gehäuse (5), welches mit dem unteren Gehäuse (2) einteilig hergestellt ist.

7. Ein Trokar (1) gemäß einem der vorangehenden Ansprüche, **gekennzeichnet durch** ein Ventil (6) umfassend ,
- einen Ventilkörper (61), welcher in Form eines Hohlzylinders ist,
- eine Stütze (62), welche es verhindert, dass das Ventil (6) sich gegen das untere Gehäuse (2) bewegt,
- eine Ventilspitze (63), welche kegelförmig ist und an dem in den Körper (5) einzudringenden Ende des Ventils (6) angeordent ist,
- eine Rille (64), welche auf der Ventilspitze (63) angeordnet ist, um den Durchgang der während der Operation zu verwendenden Instrumente zu erlauben.

8. Ein Trokar (1) gemäß Anspruch 7, **gekennzeichnet durch** die Ventilspitze (63), die aus einem nachgiebigen Material hergestellt ist.

9. Ein Trokar (1) gemäß einem der vorangehenden Ansprüche, **gekennzeichnet durch** ein Ritzel zur Luftlenkung (72), welches gegen das untere Gehäuse (2) auf dem mit dem oberen Gehäuse (5) verbundenen Teil des Luftein-und-ausgangskanals (7) gebildet ist, um es zu verhindern, dass die von dem Luftein-und -ausgangskanal geführte Luft unmittelbar auf das Ventil (6) aufschlägt.

10. Ein Trokar (1) gemäß einem der vorangehenden Ansprüche, **gekennzeichnet durch** das untere Gehäuse (2), das aus Metall bzw. Metalllegierung hergestellt ist.

11. Ein Trokar (1) gemäß einem der vorangehenden Ansprüche, **gekennzeichnet durch** das untere Gehäuse (2), das aus Polypropylen medizinischer Klasse bzw. aus Polyäthylen medizinischer Klasse hergestellt ist.

12. Ein Trokar (1) gemäß einem der vorangehenden Ansprüche, **gekennzeichnet durch** eine Ventilspitze (63), welche aus pharmazeutischem Gummi hergestellt ist.

13. Ein Trokar (1) gemäß einem der vorangehenden Ansprüche, **gekennzeichnet durch** das obere Gehäuse (5), das aus Polypropylen medizinischer Klasse bzw. aus Polyäthylen medizinischer Klasse hergestellt ist.

## Revendications

1. Un trocart utilisé dans plusieurs laparoscopique et autres chirurgies minimales invasives et par lequel passent les appareils et/ou les dispositifs qui seront utilisés pendant l'opération et/ou la chirurgie et insérés à l'intérieur du corps comportant
- au moins un corps (2) entrant à l'intérieur du corps,
- au moins un filetage (3) situé sur le corps inférieur et empêchant le corps inférieur (2) de glisser à l'intérieur du corps ou à l'extérieur du corps pendant l'opération,
- au moins un support (4) empêchant le corps (2) d'entrer complètement à l'intérieur du corps,
- au moins un corps supérieur (5) situé à la partie supérieure du corps inférieur (2) et ayant la même ou presque le même diamètre extérieur avec le corps (2) en utilisant un simple système de valves (6),
- au moins une valve (6) situé à l'intérieur du corps supérieur (5) et empêchant la fuit d'air de la cavité de corps pendant la chirurgie,
- au moins un canal d'entrée et sortie d'air (7) situé sur le corps supérieur (5) et permettant au gaz pressurisé d'être fourni à l'intérieur du corps ou aux gaz d'être libérés du corps afin de maintenir la pression du corps,
- au moins un orifice supérieur permettant aux dispositifs utilisés pendant l'opération d'être insérés dans le corps supérieur (5) et qui est situé à l'extrémité du corps supérieur (5) sur lequel se situe le corps inférieur (2),
- au moins un orifice inférieur (9) situé à l'extrémité du corps inférieur (2) entrant dans le corps et permettant aux instruments chirurgicaux d'être insérés à l'intérieur du corps supérieur (5) via l'orifice supérieur (8) et puis en avançant, au corps inférieur (2) de sortir du corps inférieur (2) et se déplacer à l'intérieur de la cavité de corps, et
- au moins une pointe de perçage (10) située à l'extrémité du corps inférieur (2) entrant dans le corps et permettant aux corps inférieur (2) d'être insérés à l'intérieur de la cavité de corps en perçant la paroi abdominale, et **caractérisé par**
- un canal d'entrée et sortie d'air (7) dont l'extrémité n'étant pas reliée au corps supérieur (5) s'étend vers le corps inférieur (2).

2. Un trocart (1) selon la revendication 1, **caractérisé par** les filetages (3) qui sont circulaires dans une façon qu'ils seront coaxiaux avec le corps inférieur (2).

3. Un trocart (1) selon l'une quelconque des revendications précédentes, **caractérisé par** les filetages (3) ayant un diamètre extérieur plus large que le filetage précédent (3) dans une manière qu'il aura un diamètre extérieur le plus petit un canal d'entrée et sortie d'air (7) à l'extrémité du corps inférieur (2) entrant dans le corps et le diamètre extérieur le plus large à l'extrémité du corps inférieur (2) relié au corps supérieur (5).

4. Un trocart (1) selon l'une quelconque des revendications précédentes, **caractérisé par** le corps inférieur (2) produit à partir d'un matériau flexible permettant aux instruments non linéaires d'être insérés à l'intérieur du corps via le trocart (1).

5. Un trocart (1) selon l'une quelconque des revendications précédentes, **caractérisé par** un support ayant une forme elliptique (4).

6. Un trocart (1) selon l'une quelconque des revendications précédentes, **caractérisé par** le corps supérieur (5) produit en une seule pièce avec le corps inférieur (2).

7. Un trocart (1) selon l'une quelconque des revendications précédentes, **caractérisé par** la valve (6) comportant
- un corps de valve (61) ayant une forme d'un cylindre creux,
- un support (62) empêchant la valve (6) de se déplacer vers le corps inférieur (2),
- une pointe de valve (6) conique et située à l'extrémité de la valve (6) étant insérée à l'intérieur du corps (5),
- une rainure (64) situé sur la pointe de valve (63) afin de permettre aux instruments qui seront utilisés pendant les chirurgies de passer.

8. Un trocart (1) selon la revendication 7, **caractérisé par** la pointe de valve (63) produite à partir d'un matériau flexible.

9. Un trocart (1) selon l'une quelconque des revendications précédentes, **caractérisé par** le biseau directeur d'air (72) qui est constitué vers le corps inférieur (2) sur le seau du canal d'entrée et sortie d'air (7) relié avec le corps supérieur (5) afin d'empêcher l'air envoyé du canal d'entrée et sortir d'air de frapper directement à la valve (6).

10. Un trocart (1) selon l'une quelconque des revendications précédentes, **caractérisé par** le corps inférieur (2) produit en métal ou alliage métallique.

11. Un trocart (1) selon l'une quelconque des revendications précédentes, **caractérisé par** le corps inférieur (2) produit à partir du polypropylène de qualité médicale ou du polyéthylène de qualité médicale.

12. Un trocart (1) selon l'une quelconque des revendications précédentes, **caractérisé par** une pointe de valve produit en caoutchouc pharmaceutique.

13. Un trocart (1) selon l'une quelconque des revendications précédentes, **caractérisé par** le corps supérieur (5) produit à partir du polypropylène de qualité médicale ou du polyéthylène de qualité médicale.
